(19) Europäisches Patentamt
European Patent Office
Office européen des brevets

(11) Veröffentlichungsnummer : **0 374 760 B1**

(12) # EUROPÄISCHE PATENTSCHRIFT

(45) Veröffentlichungstag der Patentschrift : **13.05.92 Patentblatt 92/20**

(51) Int. Cl.$^5$ : **C07C 65/03,** C07C 65/11, C07C 51/43, C07D 319/12

(21) Anmeldenummer : **89123247.2**

(22) Anmeldetag : **15.12.89**

(54) **Dioxan-Addukte aromatischer meta- oder para-Hydroxy-carbonsäuren und Verfahren zu ihrer Herstellung.**

(30) Priorität : **19.12.88 DE 3842712**

(43) Veröffentlichungstag der Anmeldung : **27.06.90 Patentblatt 90/26**

(45) Bekanntmachung des Hinweises auf die Patenterteilung : **13.05.92 Patentblatt 92/20**

(84) Benannte Vertragsstaaten : **BE CH DE FR GB IT LI NL**

(56) Entgegenhaltungen :
**EP-A- 325 925**
**EP-A- 0 341 594**

(56) Entgegenhaltungen :
**MIKROCHIM. ACTA, Band 6, 1969, Seiten 1208-1209, Univ. Innsbruck, AU; M. KUHNERT-BRANDSTÄTTER et al.:"Lösungsmitteleinschlüsse in Kristallen von p-substituierten Benzoesäurederivaten"**

(73) Patentinhaber : **HOECHST AKTIENGESELLSCHAFT**
**Postfach 80 03 20**
**W-6230 Frankfurt am Main 80 (DE)**

(72) Erfinder : **von Plessen, Helmold, Dr.**
**Kugelherrnstrasse 16**
**W-6240 Königstein/Taunus (DE)**
Erfinder : **Rittner, Siegbert, Dr.**
**Kornblumenweg 5**
**W-6082 Mörfelden-Walldorf (DE)**

**EP 0 374 760 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Als organisch-chemische Zwibchenprodukte bind eine Anzahl aromatischer Hydroxy-carbonsäuren von großer wirtschaftlicher Bedeutung. So wird z. B. 3-Hydroxy-naphthalin-2-carbonsäure in großem Umfang als Kupplungskomponente für Azofarbstoffe eingesetzt, die in der Textilfärberei verwendet werden. 2-Hydroxy-naphthalin-6-carbonsäure und 4-Hydroxybenzoesäure stellen Monomere dar, aus denen Kunststoffe und Fasern mit überragenden anwendungstechnischen Eigenschaften gewonnen werden können.

Die Herstellung solcher Hydroxy-carbonsäuren in reiner Form ist vielfach mit Schwierigkeiten verbunden. Bekannte verfahrenstechnische Grundoperationen zur Reinigung organischer Verbindungen, wie die Destillation, versagen zuweilen bei aromatischen Hydroxy-carbonsäuren, weil diese Verbindungen bei der Anwendung höherer Temperaturen intermolekular verestern oder decarboxylieren. Die Kristallisation als Reinigungsverfahren versagt ebenfalls des öfteren, weil die Verbindungen zu schwer löslich sind oder weil Verunreinigungen bei der Kristallbildung eingeschlossen werden. So kann z. B. aus den genannten Gründen 2-Hydroxy-naphthalin-6-carbonsäure nicht destilliert werden und ihre Reinigung durch Kristallisation aus Lösemitteln bietet wegen hartnäckig anhaftender Begleitsubstanzen Schwierigkeiten (EP-PS 81 753).

Die ältere, nicht vorveröffentlichte EP-PS 325925 beschreibt ein Verfahren zur Reinigung von 2-Hydroxy-naphthalin-6-carbonsäure durch Umkristallisation der Säure aus wasserlöslichen aliphatischen Ethern, Polyethern oder Hydroxy-ethern, wie 1,4-Dioxan. Ein Addukt aus 2-Hydroxy-naphthalin-6-carbonsäure und 1,4-Dioxan, das pro Mol aus 2 Mol Säure und 1 Mol Dioxan besteht, wird in der älteren, nicht vorveröffentlichten EP-OS 341 594 beschrieben. Das Addukt wird durch Kristallisation von 2-Hydroxy-naphthalin-6-carbonsäure aus Dioxan oder dessen Mischungen mit Lösemitteln hergestellt. Von M. Kuhnert-Brandstätter et al., Microchim.Acta, Band 6, 1969, S. 1208 - 1209, wird beschrieben, daß sich Einschlußverbindungen aus para-Hydroxybenzoesäure und Dioxan bilden können.

Überraschenderweise wurde nun gefunden, daß die Bildung von Addukten zwischen aromatischen Hydroxy-carbonsäuren, bei denen die Hydroxyl- und die Carboxylgruppe in metaoder para-Stellung zueinander stehen, wobei sich bei den para-Verbindungen non zusätzlich ein Fluor-, Chlor-, Brom- oder Nitrosubstituent am aromatischen Kern befindet, und 1,4-Dioxan die Möglichkeit bietet, solche Hydroxy-carbonsäuren zu reinigen. Die aus den Hydroxy-carbonsäuren und Dioxan bestehenden Addukte können darüber hinaus auch als neuartige Verbindungen oder als Zwischenprodukte verwendet werden.

Ein Gegenstand der Erfindung sind diese Addukte selbst, ein weiterer Gegenstand ist ein Verfahren zu ihrer Herstellung.

Das Verfahren zur Herstellung von Addukten aus aromatischen meta- oder para-Hydroxy-carbonsäuren und 1,4-Dioxan ist dadurch gekennzeichnet, daß man die Hydroxy-carbonsäuren, in denen die Subtituenten in meta- oder para-Stellung zueinander stehen, in 1,4-Dioxan oder dessen Mischung mit Wasser oder einem organischen Lösungsmittel löst und dann kristallisieren läßt.

Die Addukte aus den aromatischen Hydroxy-carbonsäuren und Dioxan besitzen die allgemeine Formel

$$[(R_1)\ (R_2)\ (OH)\ Ar\text{-}COOH]_2 \times [1,4\text{-Dioxan}]$$

wobei $R_1$ und $R_2$ Wasserstoff, Fluor, Chlor, Brom oder eine Nitrogruppe und Ar den Benzol- oder Naphthalinrest bedeutet. Dabei stehen die Substituenten in meta- oder para-Stellung zueinander, wobei sich beid den para-Verbindungen noch zuzätzlich ein Fluor-, Chlor-, Brom- oder Nitrosubstituent am aromatischen Kern befindet, Ihr spezielles Merkmal besteht in Wasserstoffbrückenbindungen zwischen den Hydroxygruppen der aromatischen Verbindungen und den Sauerstoffatomen des Dioxans, so daß 2 : 1-Addukte vorliegen.

Die Carboxylgruppen zweier Hydroxy-carbonsäure-moleküle sind wiederum dimerisiert, so daß sich längere kettenförmige Anordnungen bilden können.

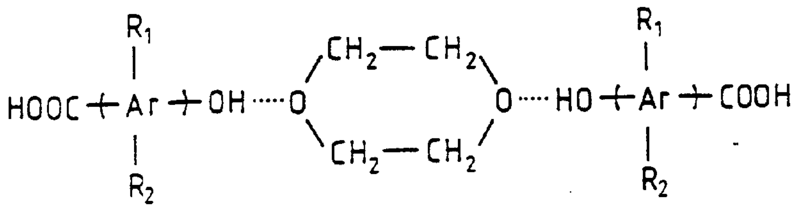

Zur Herstellung der Addukte kann man technisch gewonnene aromatische meta- oder para-Hydroxy-carbonsäuren direkt einsetzen.

Vorzugsweise löst man die Hydroxy-carbonsäuren in einer Mischung aus 1,4-Dioxan und Wasser mit einem Dioxangehalt von 20 - 90 Gew.-% unter Erwärmen und läßt die Addukte anschließend unter Abkühlen auskristallisieren. Das Kristallisat wird beispielbweise abfiltriert und unter Vakuum im Stickstoffstrom getrocknet.

Statt einer Mischung von Dioxan mit Wasser kann man auch eine Mischung von Dioxan mit einem organischen Lösungsmittel einsetzen. Man kann sogar Wasser und mit ihm mischbare organische Lösungsmittel gleichzeitig als Mischungskomponenten verwenden; beispielsweise kann man aus ternären Gemischen Dioxan/Wasser/niedere Alkohole oder Dioxan/Wasser/Polyethylenglykole das Dioxan-Addukt auskristallisieren lassen. Der Dioxangehalt der Mischungen mit Wasser und/oder organischen Lösungsmitteln beträgt im allgemeinen mindestens 10 Gew.-%, vorzugsweise mindestens 20 Gew.-%, insbesondere mindestens 30 Gew.-%.

Man kann aber auch die Hydroxy-carbonsäuren zunächst in Dioxan lösen und dann diese Lösung mit soviel Wasser oder organischen Lösungsmitteln versetzen, daß Kristallisation der Addukte eintritt. Ferner kann man aus Lösungen der Hydroxy-carbonsäuren in Dioxan oder seinen Mischungen mit Wasser oder organischen Lösungsmitteln soviel Dioxan abdestillieren, daß Kristallisation eintritt.

Die Kristallisation der Addukte kann sowohl bei Raumtemperatur als auch bei erhöhten (bis zum Siedepunkt) oder verminderten Temperaturen (bis etwa -20°C) erfolgen. Die Kristallisation ist auch bei erhöhtem Druck möglich, unter entsprechender Erhöhung des Siedepunkts.

Die Kristallisation der Addukte kann sowohl diskontinuierlich als auch kontinuierlich durchgeführt werden. Dabei kann entweder die Kühlungs- oder Verdampfungskristallisation oder die sogenannte Vakuumkristallisation angewendet werden. Hierbei kann es von Vorteil sein, unter Inertgasatmosphäre zu arbeiten. Als Inertgas kommt dabei vor allem Stickstoff in Frage. Daneben können für diesen Zweck Kohlendioxid oder Argon eingesetzt werden.

Für die erfindungsgemäße Adduktherstellung kommen insbesondere folgende Hydroxy-carbonsäuren in Frage:
3-Nitro-4-hydroxy-benzoesäure
3-Chlor-4-hydroxy-benzoesäure

Beim erfindungsgemäßen Verfahren können dem Dioxan beispielsweise folgende organische Lösungsmittel oder ihre Kombinationen zugemischt werden:
Aliphatische Kohlenwasserstoffe, etwa solche mit 5 bis 10 C-Atomen
Aromatische Kohlenwasserstoffe, wie Benzol, Toluol, Xylol, Cumol
Alicyclische Kohlenwasserstoffe, wie Cyclohexan, Dekalin, Tetralin
Chlorierte Kohlenwasserstoffe, wie $CCl_4$, $CHCl_3$, $CH_2Cl_2$, Dichlorethane, Trichlorethylen
Ester, etwa Essigsäureester, insbesondere Essigsäureethylester
Alkohole, etwa solche mit 1 bis 8 C-Atomen

Die Addukte weisen gegenüber den freien Hydroxy-carbonsäuren stark veränderte Löslichkeiten auf und lassen sich gut umkristallisieren.

Aus den erfindungsgemäßen Addukten läßt sich das Dioxan durch verschiedene chemische Operationen wieder abtrennen und so die zugrundeliegenden Hydroxycarbonsäuren in hochreiner Form zurückgewinnen.

Beispielsweise durch Erwärmung der Addukte auf etwa 100°C unter Vakuum wird das Dioxan praktisch vollständig abgespalten. In manchen Fällen ist das Dioxan im Addukt derart lose gebunden, daß bereits Temperaturen von 30 - 50°C bei Anwendung von Vakuum ausreichen, um die dioxanfreie Hydroxy-carbonsäure zurückzugewinnen.

Auch durch Umkristallisation der Addukte aus einem anderen Lösungsmittel, z. B. verdünntem Ethanol, lassen sich die dioxanfreien Hydroxy-carbonsäuren erhalten. Ferner können dioxanfreie Hydroxy-carbonsäuren durch Lösen der entsprechenden Addukte in Natriumhydroxidlösung und nachfolgende Fällung mit verdünnter Mineralsäure gewonnen werden.

Eine weitere Möglichkeit, Dioxan aus den erfindungsgemäßen Addukten abzuspalten und die zugrundeliegenden Hydroxycarbonsäuren zu gewinnen, besteht darin, das Addukt einer Wasserdampfdestillation zu unterwerfen. Man kann das Addukt auch mit einem geeigneten Lösemittel z. B. einem Keton vermischen und aus dem Gemisch Lösemittel abdestillieren, bis das Dioxan entfernt ist. Durch Abkühlung und Filtration oder Eindampfung der Lösung kann daraus die Hydroxycarbonsäure isoliert werden.

Die Adduktbildung und anschließende Freisetzung der Hydroxy-carbonsäuren ist in besonderer Weise zur Reinigung von solchen aromatischen Hydroxy-carbonsäuren geeignet, die als Vorprodukte zur Herstellung von Farbstoffen oder Kunststoffen dienen. Von besonderer Bedeutung bind beispielsweise die Addukte der 4-Hydroxy-benzoesäure und der 2-Hydroxy-naphthalin-6-carbonsäure: Die aus den beiden Addukten gewonnenen sehr reinen Hydroxy-carbonsäuren können miteinander zu sehr reinen Polyestern umgesetzt werden, aus denen man wertvolle Kunststoffe oder Fasern gewinnen kann (US-PS 4 393 191).

Die Entstehung der 2 : 1-Addukte ist überraschend, da angesichts der von den aromatischen o-Hydroxy-carbonsäuren her bekannten Wasserstoffbrücken (Chelaten)

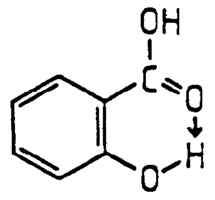

eine Ausbildung von intermolekularen Bindungen, z. B. bei p-Hydroxy-benzoesäuren, zu erwarten war.

Die in dem folgenden Beispiele genannten Prozentangaben sind Gewichtsprozente.

Beispiel

30 g 3-Nitro-4-hydroxy-benzoesäure wurden mit 100 g Dioxan und 100 g Wasser unter Erwärmen und Rühren gelöst. Die Lösung wurde mit 2,5 g Aktivkohle behandelt und anschließend heiß filtriert. Nach langsamer Abkühlung wurde das Kristallisat abfiltriert und unter Vakuum über konzentrierter Schwefelsäure getrocknet. Es wurden 28,7 g des 2 : 1-Addukts erhalten.
Röntgen-Einkristallstrukturanalyse ergab die Formel

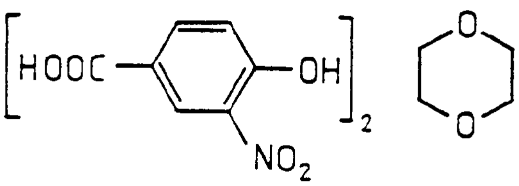

$C_{18}H_{18}N_2O_{12}$

| | |
|---|---|
| Gefunden: | C 47,3/47,4 % |
| | H 3,7/ 3,8 % |
| Berechnet: | C 47,58 % |
| | H 3,96 % |

## Patentansprüche

1. Verfahren zur Herstellung von Addukten aus aromatischen meta- oder para-Hydroxy-carbonsäuren und 1,4-Dioxan, dadurch gekennzeichnet, daß man Hydroxycarbonsäuren, in denen die Substituenten in meta- oder para-Stellung zueinander stehen, wobei sich bei den para-Verbindungen noch zusätzlich ein Fluor-, Chlor-, Brom- oder Nitrosubstituent am aromatischen Kern befindet, in 1,4-Dioxan oder dessen Mischung mit Wasser oder einem organischen Lösungsmittel löst und dann kristallisieren läßt.

2. Verfahren nach Anspruch 1, dadurch gekennzeichnet, daß man als aromatische meta- oder para-Hydroxy-carbonsäure 3-Nitro-4-hydroxy-benzoesäure oder 3-Chlor-4-hydroxy-benzoesäure einsetzt.

3. Verfahren nach Anspruch 1 oder 2, dadurch gekennzeichnet, daß man die Hydroxycarbonsäure in einer Mischung aus 1,4-Dioxan und Wasser mit einem Dioxan-Gehalt von 20 - 90 Gew.-% unter Erwärmen löst und dann unter Abkühlung auskristallisieren läßt.

4. Dioxan-Addukte von aromatischen meta- oder para-Hydroxy-carbonsäuren in denen die Substituenten in meta- oder para-Steilung zueinander stehen, wobei sich bei den para-Verbindungen noch zusätzlich ein Fluor-, Chlor-, Brom- oder Nitrosubstituent am aromatischen Kern befindet, die pro Mol aus 1 Mol 1,4-Dioxan und etwa 2 Mol Hydroxy-carbonsäure bestehen.

5. Dioxan-Addukt der 3-Nitro-4-hydroxy-benzoesäure, das pro Mol aus 1 Mol 1,4-Dioxan und etwa 2 Mol 3-Nitro-4-hydroxy-benzoesäure besteht.

6. Dioxan-Addukt der 3-Chlor-4-hydroxy-benzoesäure, das pro Mol aus 1 Mol 1,4-Dioxan und etwa 2 Mol 3-Chlor-4-hydroxy-benzoesäure besteht.

## Claims

1. A process for the preparation of an adduct of aromatic meta- or para-hydroxycarboxylic acid and 1,4-dioxane, which comprises dissolving the hydroxycarboxylic acid, in which the substituents are in the meta or para position relative to one another, in the case of the para compounds a further fluorine, chlorine, bromine or nitro substituent being situated on the aromatic nucleus, in 1,4-dioxane or a mixture of the latter with water or with an organic solvent, and then allowing the adduct to crystallize.

2. The process as claimed in claim 1, wherein the aromatic meta- or para-hydroxycarboxylic acid used is 3-nitro-4-hydroxybenzoic acid or 3-chloro-4-hydroxybenzoic acid.

3. The process as claimed in claim 1 or 2, wherein the hydroxycarboxylic acid is dissolved in a mixture of 1,4-dioxane and water having a dioxane content of 20-90% by weight, with heating, and the adduct is then allowed to crystallize out with cooling.

4. A dioxane adduct of an aromatic meta- or parahydroxycarboxylic acid, in which the substituents are in the meta or para position relative to one another, in the case of the para compounds a further fluorine, chlorine, bromine or nitro substituent being situated on the aromatic nucleus, which consists, per mole, of 1 mole of 1,4-dioxane and about 2 moles of hydroxycarboxylic acid.

5. A dioxane adduct of 3-nitro-4-hydroxybenzoic acid, which consists, per mole, of 1 mole of 1,4-dioxane and about 2 moles of 3-nitro-4-hydroxybenzoic acid.

6. A dioxane adduct of 3-chloro-4-hydroxybenzoic acid, which consists, per mole, of 1 mole of 1,4-dioxane and about 2 moles of 3-chloro-4-hydroxybenzoic acid.

## Revendications

1. Procédé pour préparer des produits d'addition dérivant d'acides m- ou p- hydroxy-carboxyliques aromatiques et du 1,4-dioxanne, procédé caractérisé en ce qu'on dissout dans du 1,4-dioxanne, ou dans un mélange de dioxanne-1,4 et d'eau ou d'un solvant organique, des acides hydroxycarboxyliques qui portent les substituants en méta ou en para l'un par rapport à l'autre et qui, dans le cas où ces substituants sont en para, portent en outre un atome de fluor, de chlore ou de brome ou un radical nitro sur le noyau aromatique, puis on laisse la cristallisation se produire.

2. Procédé selon la revendication 1, caractérisé en ce qu'on utilise, comme acide m- ou p-hydroxy-carboxylique aromatique, l'acide 3-nitro-4-hydroxy-benzoïque ou l'acide 3-chloro-4-hydroxy-benzoïque.

3. Procédé selon l'une des revendications 1 et 2, caractérisé en ce qu'on dissout, en chauffant, l'acide hydroxycarboxylique dans un mélange de 1,4-dioxanne et d'eau dont la teneur en dioxanne est comprise entre 20 et 90 % en poids, puis on laisse la cristallisation se produire tout en refroidissant.

4. Produits d'addition dérivant du dioxanne et d'acides mou p-hydroxy-carboxyliques aromatiques dans lesquels les substituants sont en position méta ou para l'un par rapport à l'autre et dans lesquels, lorsque les substituants sont en para, il y a en outre, sur le noyau aromatique, un atome de fluor, de chlore ou de brome ou un radical nitro, produits qui sont constitués, par mole, de 1 mol de 1,4-dioxanne et d'environ 2 mol d'acide hydroxy-carboxylique.

5. Produit d'addition dérivant du dioxanne et de l'acide 3-nitro-4-hydroxy-benzoïque, produit qui est constitué, par mole, de 1 mol de 1,4-dioxanne et d'environ 2 mol d'acide 3-nitro-4-hydroxy-benzoïque.

6. Produit d'addition dérivant du dioxanne et de l'acide 3-chloro-4-hydroxy-benzoïque, produit qui est constitué, par mole, de 1 mol de 1,4-dioxanne et d'environ 2 mol d'acide 3-chloro-4-hydroxy-benzoïque.